# EUROPÄISCHE PATENTSCHRIFT

(11) **EP 3 410 324 B1**
(45) Veröffentlichungstag und Bekanntmachung des Hinweises auf die Patenterteilung: **08.05.2019**
(21) Anmeldenummer: 17173349.6
(22) Anmeldetag: 30.05.2017
(51) Int. Cl.: G06F 19/00

(54) **BESTIMMEN EINES ANONYMISIERTEN DOSISBERICHTBILDES**
DETERMINING AN ANONYMOUS DOSE REPORT IMAGE
DÉTERMINATION D'UNE IMAGE DE RAPPORT DE DOSE ANONYMISÉE

(43) Veröffentlichungstag der Anmeldung: 05.12.2018
(73) Patentinhaber: Siemens Healthcare GmbH, 91052 Erlangen (DE)
(72) Erfinder: Dorn, Karlheinz, 90562 Kalchreuth (DE)

(56) Entgegenhaltungen:
- EP-A2- 1 605 348
- US-A1- 2015 128 285
- US-B1- 8 694 646

## Beschreibung

Bei bildgebenden medizinischen Untersuchungen mittels ionisierender Strahlen, insbesondere mit Röntgenstrahlen, ist eine Überwachung und Auswertung der durch den Patienten aufgenommenen Strahlendosis üblich und teilweise gesetzlich vorgeschrieben. Bei einer bildgebenden medizinischen Untersuchung mittels ionisierender Strahlung kann es sich beispielsweise um eine Röntgenaufnahme oder eine Computertomographiebildgebung handeln.

Da informationsverarbeitende Systeme für Bilddaten aus medizinischen Untersuchungen zwar Bilddaten, aber nicht immer mit Metadaten beliebiger Formate lesen können, ist ein verbreitetes Vorgehen, Metadaten über eine Untersuchung als gerasteter Text in einem Dosisberichtbild zu speichern, und das Dosisberichtbild zur Verfügung zu stellen. Bei Metadaten kann es sich insbesondere um personenbezogene Daten des untersuchten Patienten (beispielsweise Name, Alter, Geburtsdatum, Adresse und/oder Geschlecht) sowie um Dosisdaten der Untersuchung handeln, beispielsweise um einen CTDI (englisches Akronym für "Computed Tomography Dose Index", eine deutsche Übersetzung ist "Computertomographie Dosisindex"), den WED ((englisches Akronym für "water-equivalent diameter", eine deutsche Übersetzung ist "Wasseräquivalenzdurchmesser") oder das DLP (englisches Akronym für "dose length product", eine deutsche Übersetzung ist "Dosislängenprodukt"). Als Synonym für Dosisberichtbild ist auch die Bezeichnung Dosisinformationsbild bekannt.

Zur effizienten Überwachung und Auswertung der Dosisdaten ist es wünschenswert, diese Dosisberichtbilder zentral, insbesondere auch bei einem Drittanbieter für Speicherdienstleistungen (einem sogenannten "Cloudspeicher") zu speichern. Hierfür müssen aber die personenbezogenen Daten aus dem Dosisberichtbild entfernt werden oder diese anonymisiert werden, um gesetzlichen Anforderungen an den Datenschutz zu genügen.

Hierfür ist aus der Druckschrift EP 1605348 A2 eine objektspezifische Vektorisierung von gerasterten Bilddaten basierend optischer Zeichenerkennung bekannt, und aus der Druckschrift US 8,694,646 B1 sind Verfahren zur Anonymisierung von zu übermittelnden Datensätzen bekannt.

Weiterhin ist es bekannt, einzelne personenbezogene Daten mittels einer optischen Zeichenerkennung (ein englischer Fachbegriff ist "optical character recognition", kurz "OCR") zu lokalisieren und basierend auf der Position zu anonymisieren. Die Erkennung ist aber aufgrund der Variabilität der personenbezogenen Zeichen schwierig, beispielsweise kann ein Name einer Person wenig verbreitet sein oder Zeichen aus anderen Zeichensätzen (Umlaute, Akzente, arabische oder chinesische Schriftzeichen) enthalten. Weiterhin kann mit einem derartigen Verfahren nicht ausgeschlossen werden, dass im anonymisierten Dosisberichtbild weiterhin personenbezogene Daten vorhanden sind.

Weiterhin ist es aus der deutschen Patentanmeldung mit dem Aktenzeichen 10 2015 225 735.5 bekannt, Dosisberichtbilder basierend auf einer Positivliste zu verarbeiten. Hierbei werden Dosisberichtbilder übertragen, falls die enthaltenen Informationen mittels Schrifterkennung extrahiert werden können, und die entsprechende medizinische bildgebende Vorrichtung wird auf eine Positivliste übertragen. Hierbei wird aber nicht offenbart, wie personenbezogene Daten in den Dosisberichtbildern anonymisiert werden können.

Es ist daher die Aufgabe der vorliegenden Erfindung, eine Methode bereitzustellen, schnell und zuverlässig ein anonymisiertes Dosisberichtbild ohne personenbezogene Daten bereitzustellen.

Die Aufgabe wird gelöst durch ein Verfahren zum Bestimmen eines anonymisierten Dosisberichtbildes nach Anspruch 1, durch eine Bestimmungseinheit nach Anspruch 10, durch ein Computerprogrammprodukt nach Anspruch 12, sowie durch ein computerlesbares Speichermedium nach Anspruch 13.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Die Erfindung basiert darauf, dass ein personenbezogenes Dosisberichtbildes mittels einer ersten Schnittstelle empfangen wird, wobei das personenbezogene Dosisberichtbild personenbezogene Daten und Dosisdaten einer Bildgebung mittels ionisierender Strahlung umfasst, wobei die personenbezogenen Daten und die Dosisdaten als gerasterter Text im personenbezogenen Dosisberichtbild enthalten sind und wobei die personenbezogenen Daten oder die Dosisdaten ein erstes Signalwort umfassen. Weiterhin wird eine erste Position des ersten Signalwortes in dem personenbezogenen Dosisberichtbild durch optische Zeichenerkennung mittels einer ersten Recheneinheit bestimmt. Weiterhin wird ein Bildbereichs in dem personenbezogenen Dosisberichtbild basierend auf der ersten Position mittels der ersten Recheneinheit bestimmt, wobei der Bildbereich wenigstens einen Teil der personenbezogenen Daten umfasst. Weiterhin wird ein anonymisiertes Dosisberichtbild basierend auf dem personenbezogenen Dosisberichtbild mittels der ersten Recheneinheit bestimmt, wobei wenigstens der dem Bildbereich des personenbezogenen Dosisberichtbild entsprechende Bildbereich des anonymisierten Dosisberichtbildes anonymisiert wird. Insbesondere kann der vom Bildbereich umfasste Teil der personenbezogenen Daten neben dem ersten Signalwort weitere personenbezogene Daten umfassen, in anderen Worten beinhaltet der vom Bildbereich umfasste Teil der personenbezogenen Daten nicht ausschließlich das erste Signalwort.

Der Erfinder hat erkannt, dass personenbezogene Daten und Dosisdaten bei Dosisberichtbildern verschiedener Hersteller jeweils in zusammenhängenden, nicht überlappenden Bildbereichen angeordnet sind. Durch das Anonymisieren eines gesamten Bildbereiches basierend auf der Position eines Signalwortes, und nicht nur einzelner Datensätze oder des Signalwortes, können also die personenbezogenen Bilddaten besonders zuverlässig entfernt werden.

Im Gegensatz zu bekannten Verfahren ist es für das erfindungsgemäße Verfahren nicht notwendig, alle personenbezogenen Daten mittels optischer Zeichenerkennung zu finden, sondern nur einzelne Signalwörter, die eine deutlich kleinere Variabilität aufweisen als die Gesamtheit der personenbezogenen Daten im Dosisberichtbild.

Nach einem weiteren Aspekt der Erfindung wird weiterhin ein personenbezogenes Signalwort im anonymisierten Dosisberichtbild durch optische Zeichenerkennung mittels einer zweiten Recheneinheit gesucht. Hierbei kann die zweite Recheneinheit insbesondere identisch mit der ersten Recheneinheit sein. Weiterhin wird das anonymisierte Dosisberichtbildes mittels einer zweiten Schnittstelle bereitgestellt, wenn das personenbezogene Signalwort nicht im anonymisierten Dosisberichtbild enthalten ist. Hierbei kann die zweite Schnittstelle insbesondere identisch mit der ersten Schnittstelle sein. Das personenbezogene Signalwort ist vorteilhafterweise nicht mit dem ersten Signalwort identisch.

Der Erfinder hat erkannt, dass durch eine automatische Suche nach einem personenbezogenen Signalwort sichergestellt werden kann, dass keine personenbezogenen Daten im anonymisierten Dosisberichtbild vorhanden sind, ohne dass ein Dosisberichtbild manuell überprüft werden muss. Da das Bereitstellen des anonymisierten Dosisberichtbildes nur erfolgt, wenn das personenbezogene Signalwort nicht gefunden wird, kann somit sichergestellt werden, dass keine personenbezogenen Daten an einen Drittanbieter weitergegeben werden. Durch diese Verfahrensschritte kann insbesondere auch sichergestellt werden, dass keine personenbezogenen Daten weitergegeben werden, selbst wenn sich das Format des Dosisberichtbildes (beispielsweise durch Änderungen der Software oder der Hardware) ändert.

Insbesondere ist es durch ein solches Verfahren also möglich, Dosisberichtbilder automatisch zu anonymisieren und ohne personenbezogene Daten zur Verfügung zu stellen, um sie beispielsweise in Konformität mit gesetzlichen Regelungen bei einem Drittanbieter zu speichern.

Sind die erste Recheneinheit und die zweite Recheneinheit sowie die erste Schnittstelle und die zweite Schnittstelle separat ausgebildet und damit nicht identisch, so können die erste Schnittstelle und die erste Recheneinheit (z.B. als Clientrechner) insbesondere räumlich separiert von der zweiten Schnittstelle und der zweiten Recheneinheit (z.B. in der Cloud) sein. Hierdurch kann in der Cloud sichergestellt werden, dass keine personenbezogenen Daten im anonymisierten Dosisberichtbild vorhanden sind, und daher eine unautorisierte Weitergabe der personenbezogenen Daten an einen weiteren an die Cloud angeschlossenen Dienst verhindert werden. Weiterhin ist es auch möglich, das Hochladen von weiteren anonymisierten Dosisberichtbildern zu verhindern, indem beispielsweise ein Vermerk am Client, beispielsweise in Form eines Flags, gesetzt wird, und damit keine weiteren Kosten für das Hochladen von nicht ausreichend anonymisierten Dosisberichtbildern zu verursachen.

Weiterhin ist es ebenfalls möglich, die nicht ausreichend anonymisierten Dosisberichtbilder in einer Speicherumgebung mit beschränkten Zugriffsrechten zu speichern, so dass nach einer erfolgreichen Anpassung der Anonymisierungsmethode die Dosisberichtsbilder nachträglich anonymisiert werden können und damit der statistischen Auswertung zugänglich sind. Ein analoges Vorgehen in Bezug auf die Extrahierbarkeit von Daten ist in der deutschen Patentanmeldung mit dem Aktenzeichen 10 2015 225 735.5 beschrieben.

Nach einem weiteren Aspekt der Erfindung ist der Bildbereich auf genau einer Seite einer ersten Geraden durch die erste Position angeordnet, wobei die erste Gerade parallel zu einer Schriftrichtung der personenbezogenen Daten und/oder der Dosisdaten angeordnet ist. Insbesondere kann der Bildbereich durch die erste Gerade begrenzt sein, weiterhin kann der Bildbereich alle Pixel des personenbezogenen Dosisberichtbildes auf genau einer Seite der Gerade umfassen. Ist die Schriftrichtung horizontal, so ist der Bildbereich insbesondere nur über der ersten Geraden oder nur unter der ersten Geraden angeordnet. Ist die Schriftrichtung vertikal, so ist der Bildbereich insbesondere nur links oder nur rechts von der ersten Geraden angeordnet. Der Erfinder hat erkannt, dass durch eine Gerade durch die erste Position die personenbezogenen Daten besonders zuverlässig von den Dosisdaten getrennt werden können, und durch eine entsprechende Wahl des Bildbereiches die Anonymisierung besonders einfach durchführbar ist.

Nach einem weiteren Aspekt der Erfindung wird weiterhin eine zweite Position eines zweiten Signalwortes bestimmt, wobei der Bildbereich zwischen der ersten Geraden und einer zweiten Geraden durch die zweite Position angeordnet ist, wobei die zweite Gerade parallel zu der ersten Geraden ist. Insbesondere kann der Bildbereich sowohl durch die erste Gerade als auch durch die zweite Gerade begrenzt sein, weiterhin kann der Bildbereich alle Pixel des personenbezogenen Dosisberichtbildes zwischen der ersten Gerade und der zweiten Gerade umfassen. Vorteilhafterweise ist das zweite Signalwort nicht mit dem ersten Signalwort identisch. Der Erfinder hat erkannt, dass durch eine derartige Wahl des Bildbereiches besonders gut Dosisberichtbilder anonymisiert werden können, bei denen die Dosisdaten in zwei Teilbereichen angeordnet sind, die durch personenbezogene Daten getrennt sind.

Nach einem weiteren Aspekt der Erfindung weisen der Bildbereich und das personenbezogene Dosisberichtbild bezüglich einer ersten Koordinatenachse die gleiche Ausdehnung auf. Die erste Koordinatenachse ist insbesondere orthogonal zur ersten Gerade und/oder der zweiten Gerade, und damit insbesondere orthogonal zur Schriftrichtung. Der Erfinder hat erkannt, dass herstellerunabhängig in einem Dosisberichtbild bei einer horizontalen Schriftrichtung personenbezogene Daten nicht neben Dosisdaten angeordnet sind, und bei einer vertikalen Schriftrichtung personenbezogene Daten nicht über oder unter Dosisdaten angeordnet sind. Wenn also die Ausdehnung des Bildbereiches der Ausdehnung des personenbezogenen Dosisberichtbildes, können besonders zuverlässig personenbezogene Daten anonymisiert werden, ohne dass gleichzeitig fälschlicherweise Dosisdaten unkenntlich gemacht werden.

Nach einem weiteren Aspekt der Erfindung ist der Bildbereich rechteckig ausgebildet. Der Erfinder hat erkannt, dass mit einer derartigen Form des Bildbereiches besonders gut personenbezogenen Daten erfasst werden können.

Nach einem weiteren Aspekt der Erfindung umfasst der Bildbereich wenigstens zwei voneinander getrennte Teilbereiche. Der Erfinder hat erkannt, dass durch einen derartigen Bildbereich besonders gut Situationen erfasst werden können, bei denen die personenbezogenen Daten in mehreren nicht verbundenen Blöcken im personenbezogenen Dosisberichtbild enthalten sind.

Nach einem weiteren möglichen Aspekt der Erfindung wird weiterhin eine dritte Position eines dritten Signalwortes sowie eine vierte Position eines vierten Signalwortes durch optisehe Zeichenerkennung mittels der ersten Recheneinheit bestimmt. Weiterhin ist ein erster Teilbereich des Bildbereiches zwischen der ersten und einer dritten Gerade angeordnet, und ein zweiter Teilbereich des Bildbereiches ist zwischen der zweiten und einer vierten Geraden angeordnet, wobei die dritte Gerade durch die dritte Position verläuft, und wobei die vierte Gerade durch die vierte Position verläuft. Hierbei sind die dritte Gerade und/oder die vierte Gerade insbesondere parallel zu der ersten Gerade angeordnet. Vorteilhafterweise sind das dritte und das vierte Signalwort weder untereinander noch mit dem ersten oder dem zweiten Signalwort identisch. Der Erfinder hat erkannt, dass durch einen derartigen Bildbereich besonders gut Situationen erfasst werden können, bei denen die personenbezogenen Daten nicht in einem Block im personenbezogenen Dosisberichtbild enthalten sind.

Nach einem weiteren Aspekt der Erfindung wird der Bildbereich des anonymisierten Dosisberichtbild derart anonymisiert, dass aus dem Bildbereich des anonymisierten Dosisberichtbild keine der personenbezogenen Daten extrahierbar sind. In anderen Worten sind in dem Bildbereich des anonymisierten Dosisberichtbild keine personenbezogenen Daten mehr erhalten, in weiteren anderen Worten ist der Bildbereich des anonymisierten Dosisberichtbildes vollständig anonymisiert. Insbesondere wird der Bildbereich des anonymisierten Dosisberichtbildes derart anonymisiert, dass mittels optischer Zeichenerkennung keine personenbezogenen Daten aus dem Bildbereich des anonymisierten Dosisberichtbildes extrahiert werden können. Insbesondere kann jedem Pixel des Bildbereiches ein Intensitätswert zugewiesen werden, der nicht von den Intensitätswerten der Pixel des Bildbereichs im personenbezogenen Dosisberichtbild abhängt. Der Erfinder hat erkannt, dass durch eine derartige Anonymisierung der Schutz der personenbezogenen Daten besonders zuverlässig sichergestellt werden kann.

Erfindungsgemäß wird im anonymisierten Dosisberichtbild allen Pixeln des Bildbereichs des anonymisierten Dosisberichtbildes ein konstanter Wert oder ein zufälliger Wert oder ein Wert, der nicht von den Intensitätswerten der Pixel des Bildbereichs im personenbezogenen Dosisberichtbild abhängt, zugewiesen. Bei einem konstanten Wert kann es sich insbesondere um den der Hintergrundfarbe entsprechenden Wert des Dosisberichtbildes handeln, insbesondere um den maximalen oder den minimalen Wert eines Pixels. Die zufälligen Werte können insbesondere gleichverteilte Werte sein. Der Erfinder hat erkannt, dass durch eine derartige Anonymisierung alle Informationen aus dem Bildbereich entfernt werden, und somit auch besonders zuverlässig die personenbezogenen Daten entfernt werden.

Nach einem weiteren Aspekt der Erfindung ist das personenbezogene Signalwort eine Bezeichnung für einen Datensatz der personenbezogenen Daten, und das erste Signalwort ist eine Bezeichnung für einen Datensatz der Dosisdaten oder der personenbezogenen Daten. Insbesondere ist auch das zweite Signalwort, das dritte Signalwort und/oder das vierte Signalwort eine Bezeichnung für einen Datensatz der Dosisdaten oder der personenbezogenen Daten. Eine Bezeichnung eines Datensatzes kann hierbei auch als Schlüssel, als Schlüsselname oder als Name des Datensatzes bezeichnet werden. Beispielsweise ist beim Datensatz "Name = Max Mustermann" die Bezeichnung des Datensatzes "Name" (und der Wert des Datensatzes ist "Max Mustermann"), beim Datensatz "Alter: 65" ist die Bezeichnung des Datensatzes "Alter" (und der Wert des Datensatzes ist "65"). Da das personenbezogene Dosisberichtbild von einem Anwender gelesen werden muss, ist für jeden Datensatz im personenbezogenen Dosisberichtbild eine entsprechende Bezeichnung vorhanden, da ansonsten der aufgeführte Wert in der Regel nicht vom Anwender interpretiert werden kann. Es ist zu beachten, dass eine einzige Bezeichnung als Bezeichnung für mehrere Datensätze dienen kann, beispielsweise als Überschrift einer Tabelle. Der Erfinder hat erkannt, dass durch die Wahl einer Bezeichnung als Signalwort anders als bei der Suche nach dem Wert eines Datensatzes die Variabilität besonders klein ist, und daher die Position besonders zuverlässig bestimmt werden kann.

Nach einem weiteren Aspekt der Erfindung wird beim Empfangen weiterhin eine Geräteinformation empfangen, wobei das erste Signalwort, das zweite Signalwort und/oder das personenbezogene Signalwort von der Geräteinformation abhängig sind. Insbesondere können einer Geräteinformation ein erstes Signalwort, ein zweites Signalwort und/oder das personenbezogene Signalwort zugeordnet sein, insbesondere in Form einer Schlüssel-Wert-Beziehung. Hierbei identifiziert die Geräteinformation insbesondere das bildgebende medizinische Gerät, welches das Dosisdatenbild erstellt hat. Die Geräteinformation kann hierbei entweder den Typ des bildgebenden medizinischen Gerätes bezeichnen, oder das individuelle bildgebende medizinische Gerät (als Seriennummer). Der Erfinder hat erkannt, dass die Bezeichnung der Dosisdaten und/oder der personenbezogenen Daten in einem Dosisberichtbild herstellerspezifisch oder gerätespezifisch ist. Durch die Wahl beispielsweise des Signalwortes basierend auf der Geräteinformation können die personenbezogenen Daten daher besonders zuverlässig anonymisiert werden.

Die Erfindung betrifft weiterhin eine Bestimmungseinheit zum Bestimmen eines anonymisierten Dosisberichtbildes, umfassend folgende Einheiten:
- Erste Schnittstelle, ausgebildet zum Empfangen eines personenbezogenen Dosisberichtbildes,
   wobei das personenbezogene Dosisberichtbild personenbezogene Daten und Dosisdaten einer Bildgebung mittels ionisierender Strahlung umfasst, wobei die personenbezogenen Daten und die Dosisdaten als gerasterter Text im personenbezogenen Dosisberichtbild enthalten sind, und wobei die personenbezogenen Daten oder die Dosisdaten ein erstes Signalwort umfassen,
- Erste Recheneinheit, ausgebildet zum ersten Bestimmen einer ersten Position des ersten Signalwortes in dem personenbezogenen Dosisberichtbild durch optische Zeichenerkennung, weiterhin ausgebildet zum zweiten Bestimmen eines Bildbereichs in dem personenbezogenen Dosisberichtbild basierend auf der ersten Position, wobei der Bildbereich wenigstens einen Teil der personenbezogenen Daten umfasst,
weiterhin ausgebildet zum dritten Bestimmen eines anonymisierten Dosisberichtbildes basierend auf dem personenbezogenen Dosisberichtbild, wobei wenigstens der dem Bildbereich des personenbezogenen Dosisberichtbild entsprechende Bildbereich des anonymisierten Dosisberichtbildes anonymisiert ist, wobei allen Pixeln des Bildbereichs des anonymisierten Dosisberichtbild ein konstanter Wert oder ein zufälliger Wert oder ein Wert, der nicht von den Intensitätswerten der Pixel des Bildbereichs im personenbezogenen Dosisberichtbild abhängt, zugewiesen wird.

Eine solche Bestimmungseinheit kann insbesondere dazu ausgebildet sein die zuvor beschriebenen erfindungsgemäßen Verfahren und ihre Aspekte auszuführen. Die Bestimmungseinheit ist dazu ausgebildet diese Verfahren und ihre Aspekte auszuführen, indem die erste Schnittstelle und die erste Recheneinheit ausgebildet sind die entsprechenden Verfahrensschritte auszuführen. Die Bestimmungseinheit kann insbesondere auch eine zweite Recheneinheit und/oder eine zweite Schnittstelle umfassen, in diesem Fall sind auch die zweite Schnittstelle und die zweite Recheneinheit dazu ausgebildet, die entsprechenden Verfahrensschritte auszuführen.

Die Erfindung betrifft auch ein Computerprogrammprodukt mit einem Computerprogramm sowie ein computerlesbares Speichermedium. Eine weitgehend softwaremäßige Realisierung hat den Vorteil, dass auch schon bisher verwendete Bestimmungseinheiten auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Bei ionisierender Strahlung kann es sich insbesondere um Röntgenstrahlung handeln. Alternativ kann es sich auch um Alphastrahlung, Betastrahlung, Gammastrahlung oder Neutronenstrahlung handeln. Bei einer bildgebenden Untersuchung durch ionisierende Strahlung kann es sich insbesondere um eine Durchleuchtung mittels Röntgenstrahlung oder um eine Computertomographiebildebung handeln.

Ein personenbezogenes oder ein anonymisiertes Dosisberichtbild ist insbesondere eine zweidimensionale Anordnung von Pixeln, denen jeweils ein Intensitätswert zugeordnet ist. Als Synonym für "Pixel" ist auch das Wort "Bildpunkt" bekannt. Die Pixel können insbesondere auf einem rechteckigen oder einem quadratischen Gitter angeordnet sein. Die Intensitätswerte können insbesondere durch Grauwerte dargestellt sein, alternativ können die Intensitätswerte auch durch genau zwei verschiedene Farben, insbesondere durch weiß oder schwarz dargestellt sein.
Ein Signalwort ist insbesondere durch mehrere Zeichen (Buchstaben und/oder Zahlen) gegeben, insbesondere durch ein einzelnes Wort oder durch eine Gruppe von Worten. Ein Signalwort ist insbesondere die Bezeichnung eines Datensatzes. Synonyme für "Bezeichnung" des Datensatzes sind hierbei "Schlüssel", "Schlüsselname" oder "Name" des Datensatzes.

Eine Position eines Signalwortes ist durch mindestens eine Koordinate bezüglich jeweils einer Koordinatenachse gegeben. Insbesondere kann die Position eines Signalwortes auch durch eine erste Koordinate bezüglich einer ersten Koordinatenachse und durch eine zweite Koordinate bezüglich einer zweiten Koordinatenachse gegeben sein. Eine Position in einem Dosisberichtbild kann insbesondere durch Angabe genau eines Pixels gegeben sein. Die Position eines Signalwortes kann insbesondere basierend auf einem Bezugspixel des Signalwortes bestimmt werden, insbesondere kann die Position des Signalwortes aus einer Verschiebung der Koordinaten des Bezugspixels bestimmt werden. Die zur ersten Position gehörende Verschiebung kann insbesondere davon abhängen, ob die Dosisdaten oder die personenbezogenen Daten das erste Signalwort umfassen, weiterhin kann die zur zweiten Position gehörende Verschiebung davon abhängen, ob die Dosisdaten oder die personenbezogenen Daten das zweite Signalwort umfassen. Die erste Position des ersten Signalwortes muss nicht notwendigerweise durch eines der Pixel des ersten Signalwortes definiert sein oder mit diesem zusammenfallen, genauso muss die zweite Position des zweiten Signalwortes nicht notwendigerweise durch eines der Pixel des zweiten Signalwortes definiert sein oder mit diesem zusammenfallen.

Als optische Zeichenerkennung (ein englischer Fachbegriff ist "optical character recognition", kurz "OCR") wird das Extrahieren von Textinformation aus einer Rastergrafik (wie z.B. einem Bild mit Bildpunkten) bezeichnet. Als Synonym für "optische Zeichenerkennung" wird auch "Texterkennung" verwendet. Hierbei können einzelne Bildpunkte des Bildes gruppiert werden, die dann auf einen Buchstaben, eine Zahl oder ein Zeichen (die durch eine Zahl in einer Textcodierung wie ASCII oder Unicode repräsentiert werden) abgebildet werden. Optional können Methoden der Kontextanalyse (ein englischer Fachbegriff ist "intelligent character recognition", kurz "ICR") verwendet werden, um auf Wortebene oder auf Satzebene eine Fehlerkorrektur vorzunehmen, beispielsweise durch Vergleich mit einem Wörterbuch.

Die Funktionsweise von optischer Zeichenerkennung und von Kontextanalyse ist dabei dem Fachmann bekannt, daher wird für eine detailliertere Beschreibung beispielhaft auf den Artikel "A survey of modern optical character recognition techniques" von Eugene Borovikov (https://arxiv.org/abs/1412.4183v1) verwiesen. Weiterhin wird optische Zeichenerkennung durch eine Vielzahl von (teilweise frei verfügbaren) Programmpaketen zur Verfügung gestellt.

Ein Bildbereich eines personenbezogenen Dosisberichtbildes oder eines anonymisierten Dosisberichtbildes kann durch eine Menge von Pixeln des personenbezogenen Dosisberichtbildes oder eines anonymisierten Dosisberichtbildes definiert sein, wobei die Menge von Pixeln nicht notwendigerweise zusammenhängen muss. Ein Bildbereich kann auch durch eine Fläche in einem personenbezogenen Dosisberichtbild oder in einem anonymisierten Dosisberichtbild gegeben sein, insbesondere durch ein Rechteck oder durch ein Quadrat.

Zwei Geraden oder eine Gerade und eine Koordinatenachse werden als parallel bezeichnet, wenn die jeweiligen Richtungsvektoren einen Winkel von weniger als 20° oder mehr als 160°, insbesondere weniger als 10° oder mehr als 170°, insbesondere weniger als 5° oder mehr als 175°, insbesondere weniger als 1° oder mehr als 179° ausbilden. Insbesondere werden zwei Geraden oder eine Gerade und eine Koordinatenachse als parallel bezeichnet, wenn die jeweiligen Richtungsvektoren einen Winkel von 0° oder 180° ausbilden. Hierbei ist der Winkel zwischen zwei Richtungsvektoren der kleinste Winkel zwischen den Vektoren, insbesondere also immer kleiner oder gleich 180°.

Zwei Geraden oder eine Gerade und eine Koordinatenachse werden als orthogonal bezeichnet, wenn die jeweiligen Richtungsvektoren einen Winkel zwischen 70° und 110°, insbesondere zwischen 80° und 100°, insbesondere zwischen 85° und 95°, insbesondere zwischen 89° und 91° ausbilden. Insbesondere werden zwei Geraden oder eine Gerade und eine Koordinatenachse als parallel bezeichnet, wenn die jeweiligen Richtungsvektoren einen Winkel von 90° ausbilden.

Die primäre Schriftrichtung (ein Synonym ist "Schreibrichtung") eines Textes oder eines Datensatzes bezeichnet insbesondere die Richtung, in welche einzelne Buchstaben eines Wortes oder einzelne Ziffern einer Zahl ihrer Reihenfolge entsprechend aneinandergereiht werden, um das jeweilige Wort oder die jeweilige Zahl zu formen. Die primäre Schriftrichtung bei lateinischer Schrift wird als waagerecht und rechtsläufig (d.h. von links nach rechts geschrieben) bezeichnet, die Schriftrichtung im Chinesischen wird als senkrecht bezeichnet. Die Verwendung von "Schriftrichtung" ohne kennzeichnendes Adjektiv ist immer als Synonym zu "primäre Schriftrichtung" zu verstehen.

Die sekundäre Schriftrichtung eines Textes oder eines Datensatzes bezeichnet insbesondere die Richtung, in welche einzelne Zeilen eines Textes ihrer Reihenfolge entsprechend aneinandergereiht werden, um den jeweiligen Text zu formen. Die sekundäre Schriftrichtung bei lateinischer Schrift wird als senkrecht bzw. als "oben nach unten" bezeichnet. In der Regel ist die sekundäre Schriftrichtung orthogonal zur primären Schriftrichtung angeordnet.

Im Folgenden wird die Erfindung anhand der in den Figuren dargestellten Ausführungsbeispiele näher beschrieben und erläutert. Es zeigen
- Fig. 1: ein erstes Ausführungsbeispiel eines personenbezogenen Dosisberichtbildes,
- Fig. 2: ein erstes Ausführungsbeispiel eines anonymisierten Dosisberichtbildes,
- Fig. 3: ein zweites Ausführungsbeispiel eines personenbezogenen Dosisberichtbildes,
- Fig. 4: ein zweites Ausführungsbeispiel eines anonymisierten Dosisberichtbildes,
- Fig. 5: die Position eines Signalwortes,
- Fig. 6: ein Flussdiagramm eines Ausführungsbeispiels des Verfahrens,
- Fig. 7: ein erstes Ausführungsbeispiel einer Bestimmungseinheit,
- Fig. 8: ein zweites Ausführungsbeispiel einer Bestimmungseinheit.

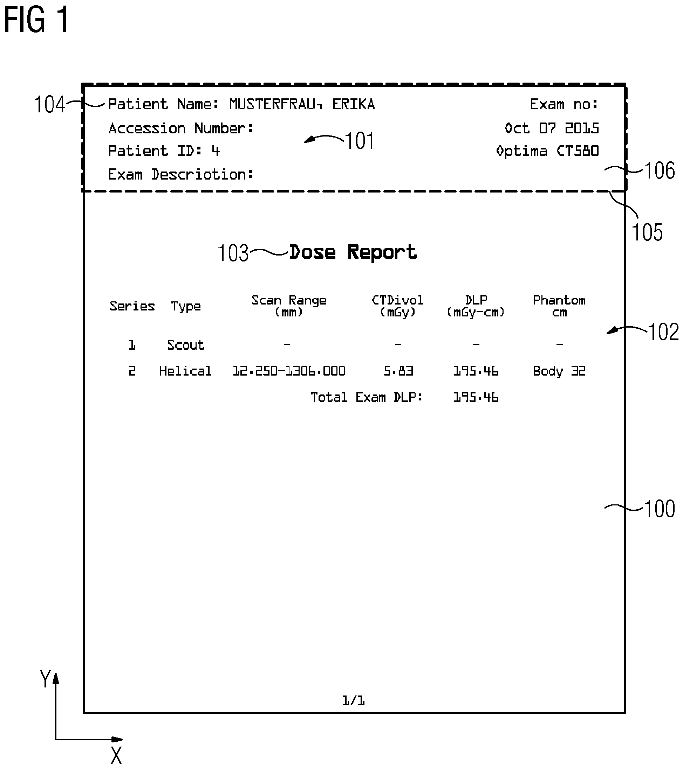

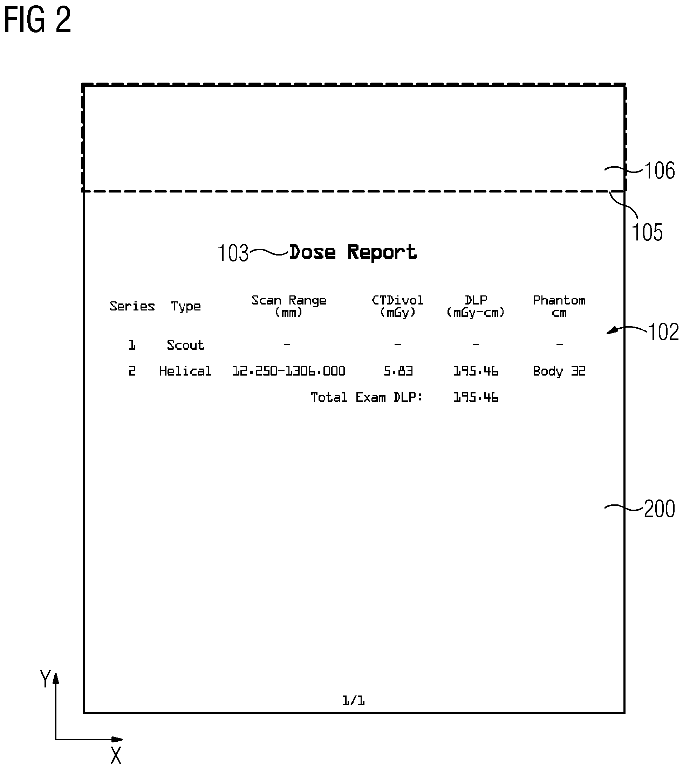

Fig. 1 zeigt ein erstes Ausführungsbeispiel eines personenbezogenen Dosisberichtbildes 100, Fig. 2 zeigt das durch das erfindungsgemäße Verfahren bestimmte zugehörige erste Ausführungsbeispiel eines anonymisierten Dosisberichtbildes 200.

Das personenbezogene Dosisberichtbild 100 umfasst personenbezogene Daten 101 und Dosisdaten 102, wobei sowohl die personenbezogene Daten 101 als Dosisdaten 102 als gerasteter Text in dem personenbezogene Dosisberichtbild 100, insbesondere in den Intensitätswerten der Pixel des personenbezogenen Dosisberichtbildes 100 enthalten sind. Die Schriftrichtung sowohl der personenbezogenen Daten 101 als auch der Dosisdaten 102 ist im dargestellten Ausführungsbeispiel horizontal, in anderen Worten parallel zu der ersten Koordinatenachse X ausgebildet. Alternativ können natürlich auch personenbezogene Dosisberichtbilder 100 mit einer oder mehreren anderen Schriftrichtungen empfangen werden.

In dem dargestellten Ausführungsbeispiel ist das erste Signalwort 103 die Wortgruppe "Dose Report". Die erste Position 504 des ersten Signalwortes 103 ist hierbei durch eine Koordinate bezüglich einer zweiten Koordinatenachse Y gegeben, insbesondere ist die erste Position 504 die um einen konstanten Wert verschobene Koordinate des Bildpunktes in der linken oberen Ecke des ersten Buchstabens "D" des ersten Signalwortes 103. Basierend auf der ersten Position 504 des ersten Signalwortes 103 kann eine erste Gerade 105 bestimmt werden, die im dargestellten Ausführungsbeispiel parallel zu der erste Koordinatenachse X angeordnet ist und durch die erste Position 504 des ersten Signalwortes 103 verläuft. In anderen Worten weisen alle Punkte der ersten Geraden 105 bezüglich der zweiten Koordinatenachse Y die gleiche Koordinate wie die erste Position 504 des ersten Signalwortes 103 auf.

Im dargestellten Ausführungsbeispiel ist der Bildbereich 106 dann durch diejenigen Pixel des personenbezogenen Dosisberichtbildes 100 gegeben, die oberhalb der ersten Gerade 105 angeordnet sind. In anderen Worten ist der Bildbereich 106 durch diejenigen Pixel des personenbezogenen Dosisberichtbildes 100 gegeben, die bezüglich der zweiten Koordinatenachse Y eine größere Koordinate aufweisen als die erste Position 504, insbesondere ist der Bildbereich 106 also durch die erste Gerade 105 begrenzt.

Das anonymisierte Dosisberichtbild 200 entspricht in diesem Ausführungsbeispiel dem personenbezogenen Dosisberichtbild 100, wobei die Intensitätswerte aller Pixel des Bildbereiches 206 im anonymisierten Dosisberichtbild 200 (der in seiner Position und in seiner Ausdehnung dem Bildbereich 106 im personenbezogenen Dosisberichtbild 100 entspricht) mit einem konstanten Wert belegt wurden. In diesem Ausführungsbeispiel entspricht der konstante Wert der Hintergrundfarbe "weiß".

Im dargestellten Ausführungsbeispiel ist das personenbezogene Signalwort 104 "Patient Name". Das personenbezogene Signalwort 104 ist, genauso wie die personenbezogenen Daten 101, im anonymisierten Dosisberichtbild 200 nicht enthalten.

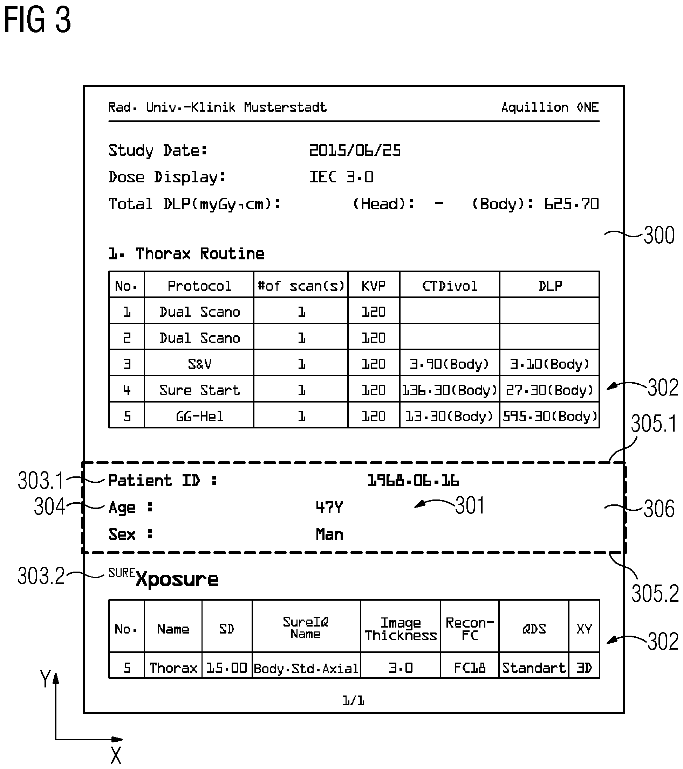

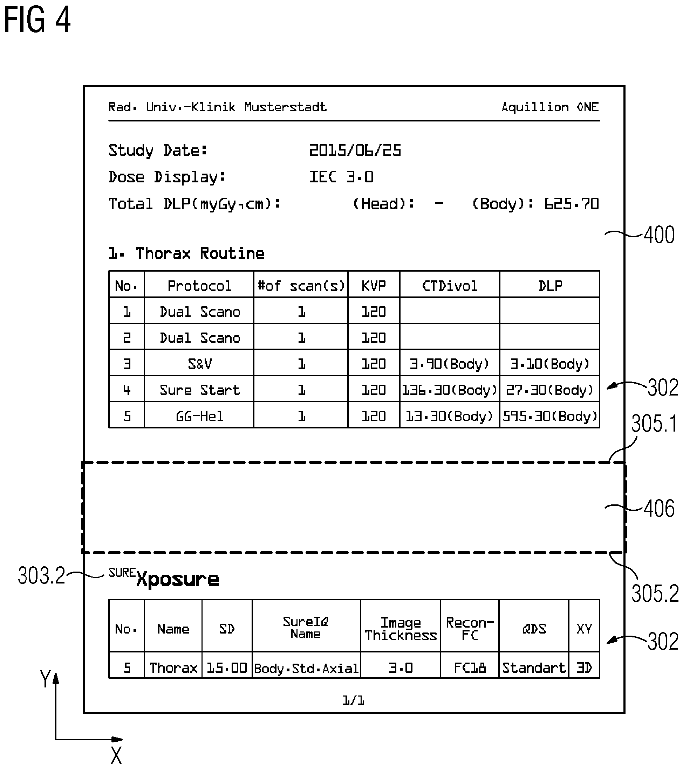

Fig. 3 zeigt ein zweites Ausführungsbeispiel eines personenbezogenen Dosisberichtbildes 300, Fig. 4 zeigt das durch das erfindungsgemäße Verfahren bestimmte zugehörige zweite Ausführungsbeispiel eines anonymisierten Dosisberichtbildes 400.

Das personenbezogene Dosisberichtbild 300 umfasst personenbezogene Daten 301 und Dosisdaten 302, wobei sowohl die personenbezogene Daten 301 als Dosisdaten 302 als gerasteter Text in dem personenbezogene Dosisberichtbild 300, insbesondere in den Intensitätswerten der Pixel des personenbezogenen Dosisberichtbildes 300 enthalten sind. Die Schriftrichtung sowohl der personenbezogenen Daten 301 als auch der Dosisdaten 302 ist im dargestellten Ausführungsbeispiel horizontal, in anderen Worten parallel zu der ersten Koordinatenachse X ausgebildet. Alternativ können natürlich auch personenbezogene Dosisberichtbilder 300 mit einer oder mehreren anderen Schriftrichtungen empfangen werden.

In dem dargestellten Ausführungsbeispiel ist das erste Signalwort 303.1 die Wortgruppe "Patient ID", das zweite Signalwort 303.2 ist das Wort "Xposure". Die erste Position 504 des ersten Signalwortes 303.1 sowie die zweite Position des zweiten Signalwortes 303.2 sind hierbei durch eine Koordinate bezüglich einer zweiten Koordinatenachse Y gegeben, insbesondere ist die erste Position 504 die um eine Konstante verschobene Koordinate des Bildpunktes in der linken oberen Ecke des ersten Buchstabens "P" des ersten Signalwortes 303.1, und die zweite Position ist die um eine Konstante verschobene Koordinate des Bildpunktes in der linken oberen Ecke des ersten Buchstabens "X" des zweiten Signalwortes 303.2. Basierend auf der ersten Position 504 des ersten Signalwortes 303.1 kann eine erste Gerade 305.1 bestimmt werden, die im dargestellten Ausführungsbeispiel parallel zu der ersten Koordinatenachse X angeordnet ist und durch die erste Position 504 des ersten Signalwortes 303.1 verläuft. In anderen Worten weisen alle Punkte der ersten Geraden 305.1 bezüglich der zweiten Koordinatenachse Y die gleiche Koordinate wie die erste Position 504 des ersten Signalwortes 303.1 auf. Weiterhin kann basierend auf der zweiten Position des zweiten Signalwortes 303.2 eine zweite Gerade 305.2 bestimmt werden, die im dargestellten Ausführungsbeispiel parallel zu der ersten Koordinatenachse Y angeordnet ist und durch die zweite Position des zweiten Signalwortes 303.2 verläuft. In anderen Worten weisen alle Punkte der zweiten Geraden 305.2 bezüglich der zweiten Koordinatenachse Y die gleiche Koordinate wie die zweite Position des zweiten Signalwortes 303.2 auf.

Im dargestellten Ausführungsbeispiel ist der Bildbereich 306 dann durch diejenigen Pixel des personenbezogenen Dosisberichtbildes 300 gegeben, die unterhalb der ersten Gerade 305.1 und gleichzeitig oberhalb der zweiten Gerade 305.2 angeordnet sind. In anderen Worten ist der Bildbereich 306 durch diejenigen Pixel des personenbezogenen Dosisberichtbildes 300 gegeben, die bezüglich der zweiten Koordinatenachse Y eine kleinere Koordinate aufweisen als die erste Position 504 und gleichzeitig eine größere Koordinate aufweisen als die zweite Position. Der Bildbereich 306 ist also insbesondere durch die erste Gerade 305.1 und die zweite Gerade 305.2 begrenzt.

Das anonymisierte Dosisberichtbild 400 entspricht in diesem Ausführungsbeispiel dem personenbezogenen Dosisberichtbild 300, wobei die Intensitätswerte aller Pixel des Bildbereiches 406 im anonymisierten Dosisberichtbild 400 (der in seiner Position und in seiner Ausdehnung dem Bildbereich 306 im personenbezogenen Dosisberichtbild 300 entspricht) mit einem konstanten Wert belegt wurden. In diesem Ausführungsbeispiel entspricht der konstante Wert der Hintergrundfarbe "weiß".

Im dargestellten Ausführungsbeispiel ist das personenbezogene Signalwort 304 "Age". Das personenbezogene Signalwort 304 ist, genauso wie die personenbezogenen Daten 301, im anonymisierten Dosisberichtbild 400 nicht enthalten.

Alternativ zu den dargestellten Ausführungsbeispielen kann der Bildbereich 106, 306 des personenbezogenen Dosisberichtbildes 100, 300 (und damit auch der Bildbereich 206, 406 des anonymisierten Dosisberichtbildes) jeweils mehrere Teilbereiche umfassen. Diese Teilbereiche können ebenfalls über Signalwörter bestimmt werden.

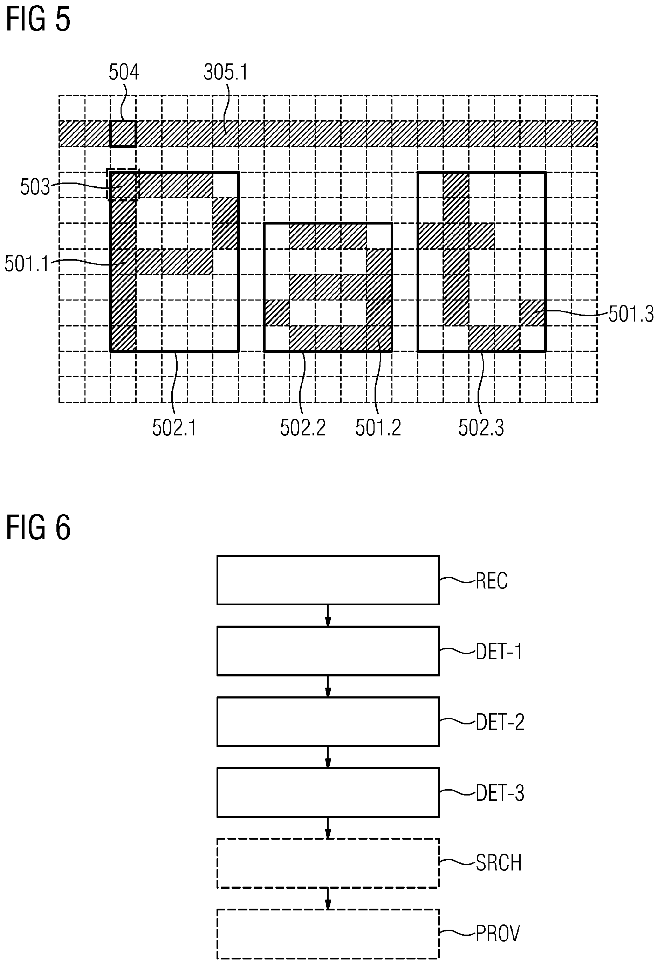

Fig. 5 zeigt eine erste Position 504 eines ersten Signalwortes 103, 303.1, die Erläuterungen zur Fig. 5 können aber auch auf die zweite Position eines zweiten Signalwortes 303.2 übertragen werden.

In der Fig. 5 sind gerasterte Buchstaben 501.1, 501.2, 501.3 des ersten Signalwortes 103, 303.1 dargestellt. Hierbei weisen die Pixel der Buchstaben 501.1, 501.2, 501.3 des ersten Signalwortes 103, 303.1 eine von der Hintergrundintensität abweichende Intensität bzw. einen vom Hintergrundgrauwert abweichenden Grauwert auf. Bekannte Algorithmen der optischen Zeichenerkennung weisen jeder Menge von Pixeln, die als zu einem einzigen Buchstaben zugehörig erkannt werden, einen Buchstabenbereich 502.1, 502.2, 502.3 zu, innerhalb dieses Buchstabenbereichs 502.1, 502.2, 502.3 findet die eigentliche Erkennung eines Buchstabens 501.1, 501.2, 501.3 statt.

Die erste Position 504 basiert auf einem der dem ersten Buchstaben 502.1 des ersten Signalwortes 103, 303.1 zugeordneten Bezugspixel 503. Der Bezugspixel 503 ist derjenige Pixel des ersten Buchstabens 501.3, der bezüglich der ersten Koordinatenachse X eine minimale Koordinate aufweist, und der bezüglich der zweiten Koordinatenachse Y eine maximale Koordinate aufweist. In anderen Worten ist der Bezugspixel 503 derjenige Pixel links oben, wenn die Horizontale des personenbezogenen Dosisberichtbildes 100, 300 mit der Schriftrichtung übereinstimmt.

In dem dargestellten Ausführungsbeispiel ist die erste Position 504 durch einen Pixel gegeben, der aus dem Bezugspixel 503 durch eine Verschiebung um zwei Pixel in die positive Richtung der zweiten Koordinatenachse Y hervorgeht. Alternativ ist es natürlich auch möglich, einen anderen Bezugspixel und/oder eine andere Verschiebung zu wählen. Der Bezugspixel und/oder die Verschiebung können insbesondere auch auf einer Geräteinformation basieren, weiterhin kann der Bezugspixel oder die Verschiebung auch davon abhängen, ob das erste Signalwort 103, 303.1 personenbezogen oder dosisbezogen ist.

Weiterhin ist in der Fig. 5 die erste Gerade 305.1 dargestellt, welche parallel zur ersten Koordinatenachse X sowie parallel zur Schriftrichtung ausgebildet ist sowie durch die erste Position 504 verläuft.

Fig. 6 zeigt ein Flussdiagramm eines Ausführungsbeispiels des Verfahrens zum Bestimmen eines anonymisierten Dosisberichtbildes 200, 400.

Der erste Schritt des dargestellten Ausführungsbeispiels ist das Empfangen REC eines personenbezogenen Dosisberichtbildes 100, 300 mittels einer ersten Schnittstelle 701, 801.1, wobei das personenbezogene Dosisberichtbild 100, 300 personenbezogene Daten 101, 301 und Dosisdaten 102, 302 einer Bildgebung mittels ionisierender Strahlung umfasst, wobei die personenbezogenen Daten 101, 301 und die Dosisdaten 102, 302 als gerasterter Text im personenbezogenen Dosisberichtbild enthalten sind, und wobei die personenbezogenen Daten 101, 301 oder die Dosisdaten 102, 302 ein erstes Signalwort 103, 303.1 umfassen.

Im dargestellten Ausführungsbeispiel ist die ionisierende Strahlung Röntgenstrahlung, und die Bildgebung ist eine Computertomographiebildgebung. Das personenbezogene Dosisberichtbild 100, 300 umfasst eine Menge von Pixeln (hier 512 mal 512 Pixel, es sind aber alternativ auch andere Bildformate möglich), denen jeweils ein Intensitätswert zugeordnet ist. In diesem Ausführungsbeispiel kann der Intensitätswert genau zwei Werte ("0" oder "1"), wobei der Wert "0" im personenbezogenen Dosisberichtbild 100, 300 schwarz dargestellt wird, und wobei der Wert "1" im personenbezogenen Dosisberichtbild 100, 300 weiß dargestellt wird. Dabei ist der Hintergrund weiß dargestellt, und der gerasterte Text ist schwarz dargestellt. Es ist alternativ auch möglich, dass der Hintergrund schwarz dargestellt ist, und der gerasterte Text weiß dargestellt ist. Alternativ sind natürlich auch Grauwerte oder Farbwerte möglich.

Die personenbezogene Daten 101, 301 und die Dosisdaten 102, 302 sind derart im personenbezogene Dosisberichtbild 100, 300 enthalten, dass sowohl die personenbezogene Daten 101, 301 als auch die Dosisdaten 102, 302 in Textform vorliegen. Der Text ist in gerasterter Form im personenbezogenen Dosisberichtbild 100, 300 enthalten, d.h. die Intensitätswerte der Pixel stellen den Text bildlich und für einen Anwender lesbar dar.

Personenbezogene Daten 101, 301 umfassen im dargestellten Ausführungsbeispiel den Namen, das Geschlecht und das Alter des mittels Computertomographie untersuchten Patienten. Dosisdaten 102, 302 umfassen beispielsweise einen CTDI (englisches Akronym für "Computed Tomography Dose Index", eine deutsche Übersetzung ist "Computertomographiedosisindex") oder das DLP (Akronym für "Dosislängenprodukt"), alternativ auch den Strom oder die Spannung der Röntgenröhre, oder andere Parameter, die zur Bewertung der während der Untersuchung im Patienten absorbierten Strahlendosis herangezogen werden können.

Es versteht sich, dass die personenbezogene Daten 101, 301 und die Dosisdaten 102, 302 noch weitere, hier nicht explizit beschriebene Parameter umfassen können.

Im dargestellten Ausführungsbeispiel umfassen die personenbezogenen Daten 101, 301 und/oder die Dosisdaten 102, 302 sowohl die Bezeichnung eines Datensatzes als auch den eigentlichen Wert des Datensatzes. Beispielsweise umfassen die personenbezogenen Daten 101, 301 als Datensatz den Namen des Patienten, dieser umfasst eine Bezeichnung ("Name:") und einen Wert ("Max Mustermann"). Die Dosisdaten 102, 302 umfassen beispielsweise den CTDI der Bildgebenden Untersuchung, dieser umfasst eine Bezeichnung ("CTDI:") und einen Wert ("10.0 mGy"). Die Position der Bezeichnung kann hierbei variieren, beispielsweise kann die Bezeichnung direkt bei dem jeweiligen Wert stehen, oder als Überschrift in einer Tabelle als Bezeichnung von mehreren Werten.

Vorteilhafterweise wird in dem dargestellten Ausführungsbeispiel beim Empfangen REC weiterhin eine Geräteinformation empfangen. Hierbei handelt es sich in diesem Ausführungsbeispiel um den Hersteller sowie die Typbezeichnung des bildgebenden medizinischen Gerätes, das das personenbezogene Dosisberichtbild erstellt hat.

Der nächste Schritt des dargestellten Ausführungsbeispiels ist das erste Bestimmen DET-1 einer ersten Position 504 des ersten Signalwortes 103, 303.1 in dem personenbezogenen Dosisberichtbild 100, 300 durch optische Zeichenerkennung mittels einer ersten Recheneinheit 702, 802.1.

Im personenbezogenen Dosisberichtbild 100 ist das erste Signalwort 103 die Wortgruppe "Dose Report", im personenbezogenen Dosisberichtbild 300 ist das erste Signalwort 303.1 die Wortgruppe "Patient ID". In einer alternativen Ausführungsform wird weiterhin die zweite Position eines zweiten Signalwortes 303.2 bestimmt. Das zweite Signalwort ist im personenbezogenen Dosisberichtbild 300 das Wort "Xposure".

Das erste Signalwort und das optionale zweite Signalwort werden im dargestellten Ausführungsbeispiel anhand der Geräteinformation aus einer Datenbank 706 abgerufen. Im dargestellten Ausführungsbeispiel umfasst die Geräteinformation eine Typbezeichnung des bildgebenden medizinischen Gerätes, eine Versionsnummer des bildgebenden medizinischen Gerätes sowie die Ausgabesprache des bildgebenden medizinischen Gerätes, wobei das bildgebende medizinische Gerät das personenbezogene Dosisberichtbild 100, 300 erstellt hat.

Alternativ zum Abrufen aus einer Datenbank ist es auch möglich, nach ein fest vorgegebenes erstes Signalwort zu verwenden. Alternativ kann auch eine Liste von vorgegebenen Signalwörtern vorgegeben sein, die nach einer gegebenen Reihenfolge abgearbeitet wird, und als erstes Signalwort das Signalwort der Liste verwendet wird, welches als erstes in dem personenbezogenen Dosisberichtbild 100, 300 durch optische Zeichenerkennung gefunden werden kann.

Im dargestellten Ausführungsbeispiel basiert die erste Position 504 auf den zweidimensionalen Koordinaten eines Bezugspixels 503 des ersten Zeichens ("D" bzw. "P") des ersten Signalwortes 103, 303.1, weiterhin basiert die zweite Position auf den zweidimensionalen Koordinaten eines Bezugspixels 503 des ersten Zeichens ("X") des zweiten Signalwortes 303.2. Insbesondere ergibt sich die erste Position 504 und die zweite Position aus der Verschiebung des jeweiligen Bezugspixels 504 in Richtung der positiven zweiten Koordinatenachse Y um einen vorgegebenen Wert, beispielsweise um zwei Pixel. Der Verschiebungswert kann optional ebenfalls in der Datenbank 706 hinterlegt sein.

Zur optischen Zeichenerkennung wird in diesem Ausführungsbeispiel das Programm "Tesseract" verwendet. Natürlich ist auch die Verwendung eines anderen Algorithmus und/oder einer anderen Software jederzeit möglich, bekannte Alternativen sind beispielsweise die Programme "Fine Reader" von ABBYY oder "Acrobat Text Capture" von Adobe.

Der nächste Schritt des dargestellten Ausführungsbeispiels ist das zweite Bestimmen DET-2 eines Bildbereichs 106, 306 in dem personenbezogenen Dosisberichtbild 100, 300 basierend auf der ersten Position 504 mittels der ersten Recheneinheit 702, 802.1, wobei der Bildbereich 106, 306 wenigstens einen Teil der personenbezogenen Daten 101, 301 umfasst. In dem dargestellten Ausführungsbeispiel weisen der Bildbereich 106, 306 und das personenbezogene Dosisberichtbild 100, 300 bezüglich der ersten Koordinatenachse X die gleiche Ausdehnung auf, alternativ sind natürlich auch andere Größen des Bildbereiches 106, 306 vorstellbar.

Hierbei wird eine erste Gerade 105, 305.1 bestimmt, wobei die erste Gerade 105, 305.1 durch die erste Position 504 verläuft und parallel zur Schriftrichtung der personenbezogenen Daten 101, 301 angeordnet ist. Optional kann auch eine zweite Gerade 305.2 bestimmt werden, die parallel zur ersten Gerade 305.1 ist und durch die zweite Position verläuft.

Für das personenbezogene Dosisberichtbild 100 ist der Bildbereich 106 bezüglich der ersten Gerade 105 auf genau einer Seite angeordnet. Insbesondere umfasst der Bildbereich 106 alle Pixel des personenbezogenen Dosisberichtbilds 100, die bezüglich der zweiten Koordinatenachse Y eine größere Koordinate aufweisen als die erste Position 504, insbesondere begrenzt die erste Gerade 105 den Bildbereich 106.

Für das personenbezogene Dosisberichtbild 300 ist der Bildbereich 306 zwischen der ersten Gerade 305.1 und der zweiten Gerade 305.2 angeordnet. Insbesondere umfasst der Bildbereich 306 alle Pixel des personenbezogenen Dosisberichtbilds 300, die bezüglich der zweiten Koordinatenachse Y eine größere Koordinate aufweisen als die zweite Position und eine kleinere Koordinate aufweisen als die erste Position 504, insbesondere begrenzen also die erste Gerade 305.1 und die zweite Gerade 305.2 den Bildbereich 306.

Die Lage des Bildbereichs 106, 306 bezüglich der ersten Position 504 und der optionalen zweiten Position bzw. bezüglich der ersten Gerade 105, 305.1 und der optionalen zweiten Gerade 305.2 kann ebenfalls in der Datenbank 706 gespeichert sein und anhand der Geräteinformation aus der Datenbank 706 abgerufen werden.

Der nächste Schritt des Verfahrens ist das dritte Bestimmen DET-3 eines anonymisierten Dosisberichtbildes 200, 400 basierend auf dem personenbezogenen Dosisberichtbild 100, 300 mittels der ersten Recheneinheit 702, 802.1, wobei wenigstens der dem Bildbereich 106, 306 des personenbezogenen Dosisberichtbild 100, 300 entsprechende Bildbereich 206, 406 des anonymisierten Dosisberichtbildes 200, 400 anonymisiert wird.

Im dargestellten Ausführungsbeispiel weist das anonymisierte Dosisberichtbild 200, 400 die gleiche Anzahl und die gleiche Anordnung von Pixeln auf wie das personenbezogene Dosisberichtbild 100, 300. Weiterhin stimmen die Intensitätswerte der Pixel des anonymisierten Dosisberichtbild 200, 400 außerhalb des Bildbereichs 206, 406 pixelweise mit den Intensitätswerten der Pixel des personenbezogenen Dosisberichtbild 100, 300 überein.

Weiterhin wird im dargestellten Ausführungsbeispiel im anonymisierten Dosisberichtbild 200, 400 jedem Pixel im Bildbereichs 306 ein konstanter Wert zugewiesen, dieser entspricht insbesondere einem dem Hintergrund zugeordneten Wert. Alternativ kann auch jedem Pixel im Bildbereich 206, 406 ein zufälliger Wert zugewiesen werden. Alternativ kann auch jedem Pixel im Bildbereich 206, 406 ein Wert zugewiesen werden, der nicht von den Werten der Pixel im Bildbereich 206, 406 im personenbezogenen Dosisberichtbild 100, 300 abhängt.

Im dargestellten Ausführungsbeispiel erfolgen als optionale nächste Schritte des Verfahrens ein Suchen SRCH eines personenbezogenen Signalworts 104, 304 im anonymisierten Dosisberichtbild 200, 400 durch optische Zeichenerkennung mittels der zweiten Recheneinheit 702, 802.2 sowie ein Bereitstellen PROV des anonymisierten Dosisberichtbildes 200, 400 mittels der zweiten Schnittstelle 701, 802.2, wenn das personenbezogene Signalwort 104, 304 nicht im anonymisierten Dosisberichtbild 200, 400 enthalten ist (das personenbezogene Signalwort 104, 304 ist in den dargestellten Ausführungsbeispielen der Fig. 2 und der Fig. 4 nicht im anonymisierten Dosisberichtbild 200, 400 enthalten, diese werden also bereitgestellt). Im dargestellten Ausführungsbeispiel ist die erste Recheneinheit 702, 802.1 mit der zweiten Recheneinheit 702, 802.2 identisch, ebenso ist die erste Schnittstelle 701, 801.1 mit der zweiten Schnittstelle 701, 801.2 identisch. Alternativ kann die erste Recheneinheit 702, 802.1 nicht mit der zweiten Recheneinheit 702, 802.2 identisch sein und/oder die erste Schnittstelle 701, 801.1 nicht mit der zweiten Schnittstelle 701, 801.2 identisch sein.

Alternativ können beim Suchen SRCH auch jederzeit mehrere personenbezogene Signalwörter gesucht werden, das Bereitstellen PROV des anonymisierten Dosisberichtbildes 200, 400 erfolgt dann, wenn keines der personenbezogenen Signalwörter im anonymisierten Dosisberichtbild 200, 400 enthalten ist. Wenn mehrere personenbezogene Signalwörter gesucht werden, kann diese Suche insbesondere parallelisiert werden, d.h. auf verschiedenen Teilen der zweiten Recheneinheit 702, 802.2 können jeweils verschiedene der personenbezogenen Signalwörter gesucht werden.

Das personenbezogene Signalwort 104, 304 oder deren Mehrzahl ist im dargestellten Ausführungsbeispiel nicht von der Geräteinformation abhängig, sondern fest vorgegeben. Es kann insbesondere die Signalwörter "Patient", "Name", "Alter" und/oder "Geschlecht" bzw. deren Übersetzung in eine andere Sprache betreffen. Alternativ ist es auch möglich, dass das personenbezogene Signalwort 104, 304 von der Geräteinformation abhängt.

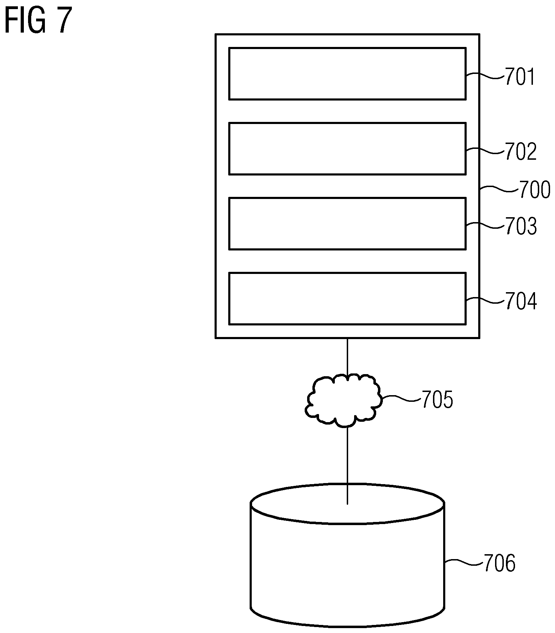

Fig. 7 zeigt ein erstes Ausführungsbeispiel einer Bestimmungseinheit 700 zur Bestimmung eines anonymisierten Dosisberichtbildes 200, 400. Die hier gezeigte Bestimmungseinheit 700 ist ausgelegt, ein erfindungsgemäßes Verfahren auszuführen. Diese Bestimmungseinheit 700 umfasst eine erste und zweite Schnittstelle 701, eine erste und zweite Recheneinheit 702, eine optionale erste Speichereinheit 703 sowie eine optionale erste Ein- und Ausgabeeinheit 704. Hierbei sind die erste und die zweite Schnittstelle 701 identisch bzw. ineinander integriert ausgebildet, weiterhin sind die erste und die zweite Recheneinheit 702 identisch bzw. ineinander integriert ausgebildet.

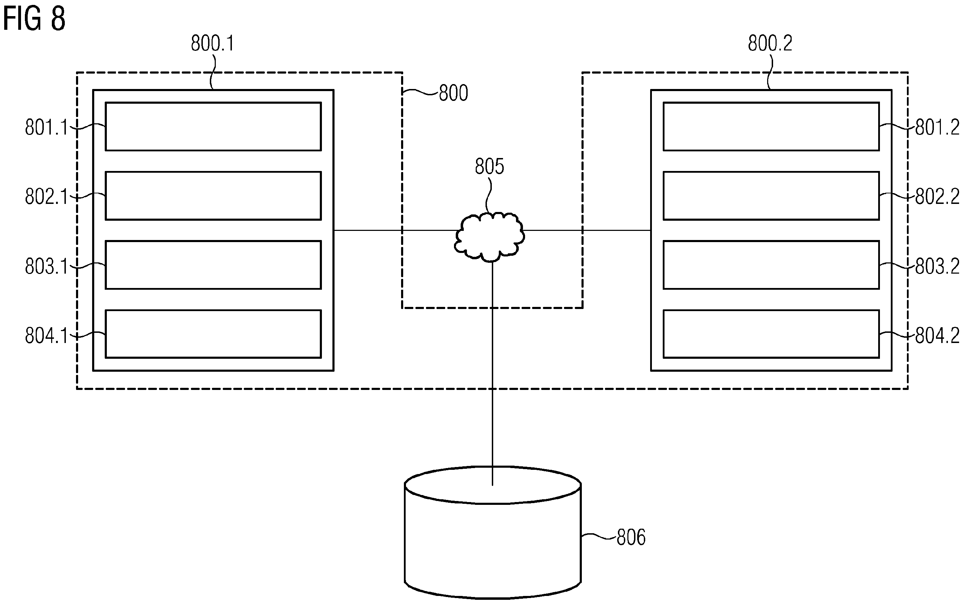

Fig. 8 zeigt ein zweites Ausführungsbeispiel einer Bestimmungseinheit 800 zur Bestimmung eines anonymisierten Dosisberichtbildes 200, 400. Die hier gezeigte Bestimmungseinheit 800 ist ausgelegt, ein erfindungsgemäßes Verfahren auszuführen. Diese Bestimmungseinheit 800 umfasst eine erste Teilbestimmungseinheit 800.1 und eine zweite Teilbestimmungseinheit 800.2, welche über ein Netzwerk 805 verbunden sind. Das Netzwerk 805 ist aber nicht notwendigerweise als Teil der Bestimmungseinheit 800 aufzufassen. Die erste Teilbestimmungseinheit 800.1 umfasst eine erste Schnittstelle 801.1, eine erste Recheneinheit 802.1, eine optionale erste Speichereinheit 803.1 sowie eine optionale erste Ein- und Ausgabeeinheit 804.1. Die zweite Teilbestimmungseinheit 800.2 umfasst eine zweite Schnittstelle 801.2, eine zweite Recheneinheit 802.2, eine optionale zweite Speichereinheit 803.2 sowie eine optionale zweite Ein- und Ausgabeeinheit 804.2.

Bei der Bestimmungseinheit 700, bei der ersten Teilbestimmungseinheit 800.1 und/oder bei der zweiten Teilbestimmungseinheit 800.2 kann es sich insbesondere um einen Computer, einen Mikrocontroller oder um einen integrierten Schaltkreis handeln. Alternativ kann es sich bei der Bestimmungseinheit 700 bei der ersten Teilbestimmungseinheit 800.1 und/oder bei der zweiten Teilbestimmungseinheit 800.2 um einen realen oder virtuellen Verbund von Computern handeln (ein englischer Fachbegriff für einen realen Verbund ist "Cluster", ein englischer Fachbegriff für einen virtuellen Verbund ist "Cloud"). Bei einer ersten Schnittstelle 701, 801.1 und/oder bei einer zweiten Schnittstelle 701, 801.2 kann es sich um eine Hardware- oder Softwareschnittstelle handeln (beispielsweise PCI-Bus, USB oder Firewire). Eine erste Schnittstelle 701, 801.1 und/oder eine zweite Schnittstelle 701, 801.2 kann insbesondere auch einzelne Teilschnittstellen umfassen, insbesondere eine Eingabeschnittstelle und/oder eine Ausgabeschnittstelle. Eine erste Recheneinheit 702, 802.1 und/oder eine zweite Recheneinheit 702, 802.2 kann Hardware-Elemente oder Software-Elemente aufweisen, beispielsweise einen Mikroprozessor oder ein sogenanntes FPGA (englisches Akronym für "Field Programmable Gate Array"). Eine erste Speichereinheit 703, 803.1 und/oder eine zweite Speichereinheit 803.2 kann als nicht dauerhafte Arbeitsspeicher (Random Access Memory, kurz RAM) oder als dauerhafter Massenspeicher (Festplatte, USB-Stick, SD-Karte, Solid State Disk) realisiert sein. Eine erste Ein- und Ausgabeeinheit 704, 804.1 und/oder eine zweite Ein- und Ausgabeeinheit 804.2 umfasst wenigstens eine Eingabeeinheit und/oder wenigstens eine Ausgabeeinheit.

In den dargestellten Ausführungsbeispielen ist die Bestimmungseinheit 700 bzw. die erste Teilbestimmungseinheit 800.1 und die zweite Teilbestimmungseinheit 800.2 über ein Netzwerk 705, 805 mit einer Datenbank 706, 806 verbunden. Bei einem Netzwerk 705, 805 kann es sich um ein kabelgebundenes Netzwerk (beispielsweise Ethernet oder USB) oder um ein kabelloses Netzwerk (beispielweise über Bluetooth oder ein drahtloses Netzwerk wie "Wireless LAN"). Beim Netzwerk 705, 805 kann es sich auch um ein Intranet oder das Internet handeln. Die Datenbank 706, 806 ist im dargestellten Beispiel separat von der Bestimmungseinheit 700, 800 in einem Datenbankserver ausgeführt, alternativ kann die Datenbank 706, 806 auch in der ersten Speichereinheit 703, 803.1 und/oder der zweiten Speichereinheit 803.2 der Bestimmungseinheit 700, 800 ausgeführt sein. Die Verbindung der Bestimmungseinheit 700, 800 mit der Datenbank 706, 806 ist optional. Es ist im zweiten Ausführungsbeispiel auch möglich, dass sowohl die erste Teilbestimmungseinheit 800.1 als auch die zweite Teilbestimmungseinheit 800.2 mit einer separaten Datenbank über ein separates Netzwerk verbunden sind, und dass diese separaten Netzwerke nicht mit dem Netzwerk 805 zur Verbindung der ersten Teilbestimmungseinheit 800.1 und der zweiten Teilbestimmungseinheit 800.2 identisch ist.

Die Datenbank 706, 806 umfasst in den dargestellten Ausführungsbeispielen Zuordnungen zwischen Geräteinformationen von bildgebenden medizinischen Geräten und Informationen zur Ausführung des erfindungsgemäßen Verfahrens. Hierbei kann die Geräteinformation insbesondere den Hersteller, die Typbezeichnung und/oder eine Seriennummer des bildgebenden medizinischen Gerätes umfassen. Informationen zur Ausführung des erfindungsgemäßen Verfahrens können eine oder mehrere der folgenden Informationen umfassen:
- Erstes Signalwort 103.1, 303.1, zweites Signalwort 303.2 und/oder personenbezogenes Signalwort 104, 304
- Abhängigkeit der ersten Position 504 von der Position der Pixel des ersten Signalwortes 103.1, 303.1 und/oder Abhängigkeit der zweiten Position von der Position der Pixel des zweiten Signalwortes 303.2
- Lage und Ausdehnung des Bildbereichs 106, 306 relativ zur ersten Position 504 und/oder relativ zur zweiten Position
Diese Informationen müssen aber nicht notwendigerweise in der Datenbank 706, 806 gespeichert sein, sondern können beispielsweise auch fest in einem Programmcode vorgegeben sein.

## Patentansprüche

1. Verfahren zum Bestimmen eines anonymisierten Dosisberichtbildes (200, 400), umfassend folgende Verfahrensschritte:
- Empfangen (REC) eines personenbezogenen Dosisberichtbildes (100, 300) mittels einer ersten Schnittstelle (701, 801.1), wobei das personenbezogene Dosisberichtbild (100, 300) personenbezogene Daten (101, 301) und Dosisdaten (102, 302) einer Bildgebung mittels ionisierender Strahlung umfasst,
wobei die personenbezogenen Daten (101, 301) und die Dosisdaten (102, 302) als gerasterter Text im personenbezogenen Dosisberichtbild enthalten sind,
und wobei die personenbezogenen Daten (101, 301) oder die Dosisdaten (102, 302) ein erstes Signalwort (103, 303.1) umfassen,
- Erstes Bestimmen (DET-1) einer ersten Position (504) des ersten Signalwortes (103, 303.1) in dem personenbezogenen Dosisberichtbild (100, 300) durch optische Zeichenerkennung mittels einer ersten Recheneinheit (702, 802.1),
- Zweites Bestimmen (DET-2) eines Bildbereichs (106, 306) in dem personenbezogenen Dosisberichtbild (100, 300) basierend auf der ersten Position (504) mittels der ersten Recheneinheit (702, 802.1),
wobei der Bildbereich (106, 306) wenigstens einen Teil der personenbezogenen Daten (101, 301) umfasst,
- Drittes Bestimmen (DET-3) eines anonymisierten Dosisberichtbildes (200, 400) basierend auf dem personenbezogenen Dosisberichtbild (100, 300) mittels der ersten Recheneinheit (702, 802.1),
wobei wenigstens der dem Bildbereich (106, 306) des personenbezogenen Dosisberichtbild (100, 300) entsprechende Bildbereich (206, 406) des anonymisierten Dosisberichtbildes (200, 400) anonymisiert wird,
wobei allen Pixeln des Bildbereichs (206, 406) des anonymisierten Dosisberichtbild (200, 400) ein konstanter Wert oder ein zufälliger Wert oder ein Wert, der nicht von den Intensitätswerten der Pixel des Bildbereichs (106, 306) im personenbezogenen Dosisberichtbild (100, 300) abhängt, zugewiesen wird.

2. Verfahren nach dem Anspruch 1, weiterhin umfassend folgende Verfahrensschritte:
- Suchen (SRCH) eines personenbezogenen Signalworts (104, 304) im anonymisierten Dosisberichtbild (200, 400) durch optische Zeichenerkennung mittels einer zweiten Recheneinheit (702, 802.2),
- Bereitstellen (PROV) des anonymisierten Dosisberichtbildes (200, 400) mittels einer zweiten Schnittstelle (701, 801.2), wenn das personenbezogene Signalwort (104, 304) nicht im anonymisierten Dosisberichtbild (200, 400) enthalten ist.

3. Verfahren nach einem der vorherigen Ansprüche, wobei der Bildbereich (106, 306) auf genau einer Seite einer ersten Geraden (105, 305.1) durch die erste Position (504) angeordnet ist,
wobei die erste Gerade (105, 305.1) parallel zu einer Schriftrichtung der personenbezogenen Daten und/oder der Dosisdaten angeordnet ist.

4. Verfahren nach dem Anspruch 3, wobei beim ersten Bestimmen (DET-1) weiterhin eine zweite Position eines zweiten Signalwortes (305.2) bestimmt wird,
wobei der Bildbereich (306) zwischen der ersten Geraden (303.1) und einer zweiten Geraden (303.2) durch die zweite Position angeordnet ist,
wobei die zweite Gerade (303.2) parallel zu der ersten Geraden (303.1) ist.

5. Verfahren nach Anspruch 3 oder 4, wobei der Bildbereich (106, 306) und das personenbezogene Dosisberichtbild (100, 300) entlang einer ersten Koordinatenachse (X) die gleiche Ausdehnung aufweisen.

6. Verfahren nach einem der vorherigen Ansprüche, wobei der Bildbereich (106, 306) rechteckig ausgebildet ist.

7. Verfahren nach einem der vorherigen Ansprüche, wobei der Bildbereich (106, 306) wenigstens zwei voneinander getrennte Teilbereiche umfasst.

8. Verfahren nach einem der vorherigen Ansprüche, wobei das personenbezogene Signalwort (104, 304) eine Bezeichnung für einen Datensatz der personenbezogenen Daten (101, 301) ist, und/oder wobei das erste Signalwort (103, 303.1) eine Bezeichnung für einen Datensatz der Dosisdaten (102, 302) oder der personenbezogenen Daten (101, 301) ist.

9. Verfahren nach einem der vorherigen Ansprüche, wobei beim Empfangen (REC) weiterhin eine Geräteinformation empfangen wird,
wobei das erste Signalwort (103, 303.1), das zweite Signalwort (303.2) und/oder das personenbezogene Signalwort (104, 304) von der Geräteinformation abhängig sind.

10. Bestimmungseinheit (700, 800) zum Bestimmen eines anonymisierten Dosisberichtbildes (200, 400), umfassend folgende Einheiten:
- Erste Schnittstelle (701, 801.1), ausgebildet zum Empfangen (REC) eines personenbezogenen Dosisberichtbildes (100, 300), wobei das personenbezogene Dosisberichtbild (100, 300) personenbezogene Daten (101, 301) und Dosisdaten (102, 302) einer Bildgebung mittels ionisierender Strahlung umfasst, wobei die personenbezogenen Daten (101, 301) und die Dosisdaten (102, 302) als gerasterter Text im personenbezogenen Dosisberichtbild (100, 300) enthalten sind, und wobei die personenbezogenen Daten (101, 301) oder die Dosisdaten (102, 302) ein erstes Signalwort (103, 303.1) umfassen,
- Erste Recheneinheit (702, 802.1), ausgebildet zum ersten Bestimmen (DET-1) einer ersten Position (504) des ersten Signalwortes (103, 303.1) in dem personenbezogenen Dosisberichtbild (100, 300) durch optische Zeichenerkennung, weiterhin ausgebildet zum zweiten Bestimmen (DET-2) eines Bildbereichs (106, 306) in dem personenbezogenen Dosisberichtbild (100, 300) basierend auf der ersten Position (504), wobei der Bildbereich (106, 306) wenigstens einen Teil der personenbezogenen Daten (101, 301) umfasst,
weiterhin ausgebildet zum dritten Bestimmen (DET-3) eines anonymisierten Dosisberichtbildes (200, 400) basierend auf dem personenbezogenen Dosisberichtbild (100, 300), wobei wenigstens der dem Bildbereich (106, 306) des personenbezogenen Dosisberichtbild (100, 300) entsprechende Bildbereich (206, 406) des anonymisierten Dosisberichtbildes (200, 400) anonymisiert wird, wobei allen Pixeln des Bildbereichs (206, 406) des anonymisierten Dosisberichtbild (200, 400) ein konstanter Wert oder ein zufälliger Wert oder ein Wert, der nicht von den Intensitätswerten der Pixel des Bildbereichs (106, 306) im personenbezogenen Dosisberichtbild (100, 300) abhängt, zugewiesen wird.

11. Bestimmungseinheit (700, 800) nach Anspruch 10, weiterhin ausgebildet ein Verfahren nach einem der Ansprüche 2 bis 9 auszuführen.

12. Computerprogrammprodukt mit einem Computerprogramm, welches direkt in einen Speicher (703, 803.1, 803.2) einer Bestimmungseinheit (700, 800) ladbar ist, mit Programmabschnitten, um alle Schritte des Verfahrens nach einem der Ansprüche 1 bis 9 auszuführen, wenn die Programmabschnitte von der Bestimmungseinheit (700, 800) ausgeführt werden.

13. Computerlesbares Speichermedium, auf welchem von einer Bestimmungseinheit (700, 800) lesbare und ausführbare Programmabschnitte gespeichert sind, um alle Schritte des Verfahrens nach einem der Ansprüche 1 bis 9 auszuführen, wenn die Programmabschnitte von der Bestimmungseinheit (700, 800) ausgeführt werden.

## Claims

1. Method for determining an anonymised dose report image (200, 400), comprising the following method steps:
- Receipt (REC) of a personal dose report image (100, 300) by means of a first interface (701, 801.1),
wherein the personal dose report image (100, 300) comprises personal data (101, 301) and dose data (102, 302) of an imaging by means of ionising radiation,
wherein the personal data (101, 301) and the dose data (102, 302) are contained as rasterised text in the personal dose report image,
and wherein the personal data (101, 301) or the dose data (102, 302) comprise a first signal word (103, 303.1),
- First determination (DET-1) of a first position (504) of the first signal word (103, 303.1) in the personal dose report image (100, 300) through optical character recognition by means of a first computing unit (702, 802.1),
- Second determination (DET-2) of an image area (106, 306) in the personal dose report image (100, 300) based on the first position (504) by means of the first computing unit (702, 802.1),
wherein the image area (106, 306) comprises at least a part of the personal data (101, 301),
- Third determination (DET-3) of an anonymised dose report image (200, 400) based on the personal dose report image (100, 300) by means of the first computing unit (702, 802.1), wherein at least the image area (206, 406) of the anonymised dose report image (200, 400) corresponding to the image area (106, 306) of the personal dose report image (100, 300) is anonymised,
wherein all pixels of the image area (206, 406) of the anonymised dose report image (200, 400) are allocated a constant value or a random value or a value which does not depend on the intensity values of the pixels of the image area (106, 306) in the personal dose report image (100, 300).

2. Method according to claim 1, further comprising the following method steps:
- Searching (SRCH) for a personal signal word (104, 304) in the anonymised dose report image (200, 400) through optical character recognition by means of a second computing unit (702, 802.2),
- Provision (PROV) of the anonymised dose report image (200, 400) by means of a second interface (701, 801.2), if the personal signal word (104, 304) is not contained in the anonymised dose report image (200, 400).

3. Method according to one of the preceding claims, wherein the image area (106, 306) is arranged on precisely one side of a first straight line (105, 305.1) through the first position (504),
wherein the first straight line (105, 305.1) is arranged in parallel to a text direction of the personal data and/or of the dose data.

4. Method according to claim 3, wherein during first determination (DET-1), a second position of a second signal word (305.2) is further determined,
wherein the image area (306) is arranged between the first straight line (303.1) and a second straight line (303.2) through the second position,
wherein the second straight line (303.2) is parallel to the first straight line (303.1).

5. Method according to claim 3 or 4, wherein the image area (106, 306) and the personal dose report image (100, 300) have the same extent along a first coordinate axis (X).

6. Method according to one of the preceding claims, wherein the image area (106, 306) is embodied as a rectangle.

7. Method according to one of the preceding claims, wherein the image area (106, 306) comprises at least two subareas separated from one another.

8. Method according to one of the preceding claims, wherein the personal signal word (104, 304) is a designation for a dataset of the personal data (101, 301), and/or wherein the first signal word (103, 303.1) is a designation for a dataset of the dose data (102, 302) or of the personal data (101, 301).

9. Method according to one of the preceding claims, wherein, during receipt (REC), device information is also received, wherein the first signal word (103, 303.1), the second signal word (303.2) and/or the personal signal word (104, 304) are dependent on the device information.

10. Determination unit (700, 800) for determining an anonymised dose report image (200, 400), comprising the following units:
- First interface (701, 801.1), embodied for receiving (REC) a personal dose report image (100, 300),
wherein the personal dose report image (100, 300) comprises personal data (101, 301) and dose data (102, 302) of an imaging by means of ionising radiation, wherein the personal data (101, 301) and the dose data (102, 302) are contained as rasterised text in the personal dose report image (100, 300), and wherein the personal data (101, 301) or the dose data (102, 302) comprise a first signal word (103, 303.1),
- First computing unit (702, 802.1), embodied for first determination (DET-1) of a first position (504) of the first signal word (103, 303.1) in the personal dose report image (100, 300) through optical character recognition,
further embodied for second determination (DET-2) of an image area (106, 306) in the personal dose report image (100, 300) based on the first position (504), wherein the image area (106, 306) comprises at least a part of the personal data (101, 301),
further embodied for third determination (DET-3) of an anonymised dose report image (200, 400) based on the personal dose report image (100, 300), wherein at least the image area (206, 406) of the anonymised dose report image (200, 400) corresponding to the image area (106, 306) of the personal dose report image (100, 300) is anonymised, wherein all pixels of the image area (206, 406) of the anonymised dose report image (200, 400) are allocated a constant value or a random value or a value which does not depend on the intensity values of the pixels of the image area (106, 306) in the personal dose report image (100, 300).

11. Determination unit (700, 800) according to claim 10, further embodied to carry out a method according to one of claims 2 to 9.

12. Computer program product with a computer program, which is able to be loaded directly into a memory (703, 803.1, 803.2) of a determination unit (700, 800), with program sections for carrying out all the steps of the method according to one of claims 1 to 9, when the program sections are executed by the determination unit (700, 800).

13. Computer-readable storage medium, on which program sections able to be read and executed by a determination unit (700, 800) are stored, in order to execute all steps of the method according to one of claims 1 to 9, when the program sections are executed by the determination unit (700, 800).

## Revendications

1. Procédé de détermination d'une image (200, 400) de rapport de dose anonymisée, comprenant les stades de procédé suivants :
- réception (REC) d'une image (100, 300) de rapport de dose rapportée à la personne au moyen d'une première interface (701, 801.1), l'image (100, 300) de rapport de dose rapportée à la personne comprenant des données (101, 301) rapportées à la personne et des données (102, 302) de dose d'une imagerie au moyen d'un rayonnement ionisé,
dans lequel les données (101, 301) rapportées à la personne et les données (102, 302) de dose sont contenues sous la forme d'un texte tramé dans l'image de rapport de dose rapportée à la personne,
et dans lequel les données (101, 301) rapportées à la personne ou les données (102, 302) de dose comprennent un premier mot (103, 303.1) de signal,
- première détermination (DET-1) d'une première position (504) du premier mot (103, 303.1) de signal dans l'image (100, 300) de rapport de dose rapportée à la personne par détection optique de caractères au moyen d'une première unité (702, 802.1) informatique,
- deuxième détermination (DET-2) d'une partie (106, 306) de l'image (100, 300) de dose rapportée à la personne reposant sur la première position (504) au moyen de la première unité (702, 802.1) informatique,
dans lequel la partie (106, 306) d'image comprend au moins une partie des données (101, 301) rapportées à la personne,
- troisième détermination (DET-3) d'un rapport (200, 400) d'image de dose anonymisée reposant sur l'image (100, 300) de rapport de dose rapportée à la personne au moyen de la première unité (702, 802.1) informatique,
dans lequel on anonymise au moins la partie (206, 406) de l'image (200, 400) de rapport de dose anoymisée correspondant à la partie (106, 306) de l'image (100, 300) de rapport de dose rapportée à la personne,
dans lequel on associe, à tous les pixels de la partie (206, 406) de l'image (200, 400) de rapport de dose anonymisée, une valeur constante ou une valeur aléatoire ou une valeur qui ne dépend pas des valeurs d'intensité des pixels de la partie (106, 306) de l'image (100, 300) de rapport de dose rapportée à la personne.

2. Procédé suivant la revendication 1, comprenant, en outre, les stades de procédé suivants :
- recherche (SRCH) d'un mot (104, 304) de signal rapporté à la personne dans l'image (200, 400) de rapport de dose anonymisée par détection optique de caractères au moyen d'une deuxième unité (702, 802.2) informatique,
- mise à disposition (PROV) de l'image (200, 400) de rapport de dose anonymisée au moyen d'une deuxième interface (701, 801.2), si le mot (104, 304) de signal rapporté à la personne n'est pas contenu dans l'image (200, 400) de rapport de dose anonymisée.

3. Procédé suivant l'une des revendications précédentes, dans lequel la partie (106, 306) d'image est, par la première position (504), mise exactement d'un côté d'une première droite (105, 305.1),
dans lequel la première droite (105, 305.1) est parallèle à une direction d'écriture des données rapportées à la personne et/ou des données de dose.

4. Procédé suivant la revendication 3, dans lequel, lors de la première détermination (DET-a), on détermine, en outre, une deuxième position d'un deuxième mot (305.2) de signal,
dans lequel la partie (306) d'image est mise, par la deuxième position, entre la première droite (303.1) et une deuxième droite (303.2),
dans lequel la deuxième droite (303.2) est parallèle à la première droite (303.1).

5. Procédé suivant la revendication 3 ou 4, dans lequel la partie (106, 306) d'image et l'image (100, 300) de rapport de dose rapportée à la personne ont la même étendue suivant un premier axe (X) de coordonnées.

6. Procédé suivant l'une des revendications précédentes, dans lequel la partie (106, 306) d'image est rectangulaire.

7. Procédé suivant l'une des revendications précédentes, dans lequel la partie (106, 306) d'image comprend au moins deux sous-parties distinctes l'une de l'autre.

8. Procédé suivant l'une des revendications précédentes, dans lequel le mot (104, 304) de signal rapporté à la présente est une désignation d'un jeu des données (101, 301) rapportées à la personne et/ou dans lequel le premier mot (103, 303.1) de signal est une désignation d'un jeu des données (102, 302) de dose ou des données (101, 301) rapportées à la personne.

9. Procédé suivant l'une des revendications précédentes, dans lequel, lors de la réception (REC), on reçoit, en outre, une information d'appareil,
dans lequel le premier mot (103, 303.1) de signal, le deuxième mot (303.2) de signal et/ou le mot (104, 304) de signal rapporté à la personne sont indépendants de l'information d'appareil.

10. Unité (700, 800) de détermination pour déterminer une image (200, 400) de rapport de dose anonymisée, comprenant les unités suivantes :
- une première interface (701, 801.1) constituée pour recevoir (REC) une image (100, 300) de rapport de dose rapportée à la personne, l'image (100, 300) de rapport de dose rapportée à la personne comprenant des données (101, 301) rapportées à la personne et des données (102, 302) de dose d'une imagerie au moyen d'un rayonnement ionisé, les données (101, 301) rapportées à la personne et les données (102, 302) de dose étant contenues sous la forme d'un texte tramé dans l'image (100, 300) de dose rapportée à la personne et les données (101, 301) rapportées à la personne ou les données (102, 302) de dose comprenant un premier mot (103, 303.1) de signal,
- une première unité (702, 802.1) informatique constituée pour la première détermination (DET-1) d'une première position (504) du premier mot (103, 303.1) de signal dans l'image (100, 300) de rapport de dose rapportée à la personne par détection optique de caractères, constituée, en outre, pour la deuxième détermination (DET-2) d'une partie (106, 306) de l'image (100, 300) de rapport de dose rapportée à la personne sur la base de la première position (504), la partie (106, 306) d'image comprenant au moins une partie des données (101, 301) rapportées à la personne, constituée, en outre, pour la troisième détermination (DET-3), d'une image (200, 400) de rapport de dose anonymisée reposant sur l'image (100, 300) de rapport de dose rapportée à la personne, au moins la partie (206, 406) d'image correspondant à la partie (106, 306) de l'image (100, 300) de rapport de dose rapportée à la personne, de l'image (200, 400) de rapport de dose anonymisée, étant anonymisée, dans lequel on associe, à tous les pixels de la partie (206, 406) de l'image (200, 400) de rapport de dose anonymisée, une valeur constante ou une valeur aléatoire ou une valeur qui ne dépend pas des valeurs d'intensité des pixels de la partie (106, 306) de l'image (100, 300) de rapport de dose rapportée à la personne.

11. Unité (700, 800) de détermination suivant la revendication 10, constituée, en outre, pour exécuter un procédé suivant l'une des revendications 2 à 9.

12. Produit de programme d'ordinateur, comprenant un programme d'ordinateur, qui peut être chargé directement dans une mémoire (703, 803.1, 803.2) d'une unité (700, 800) de détermination, ayant des parties de programme pour effectuer tous les stades du procédé suivant l'une des revendications 1 à 9, lorsque les parties du programme sont exécutées par l'unité (700, 800) de détermination.

13. Support de mémoire déchiffrable par ordinateur, sur lequel sont mémorisées des parties de programme pouvant être déchiffrées et réalisées par une unité (700, 800) de détermination, afin d'effectuer tous les stades du procédé suivant l'une des revendications 1 à 9, lorsque les parties de programme sont exécutées par l'unité (700, 800) de détermination.
